# FASCICULE DE BREVET EUROPEEN

(11) **EP 1 824 857 B1**
(45) Date de publication et mention de la délivrance du brevet: **01.12.2010**
(21) Numéro de dépôt: 05824656.2
(22) Date de dépôt: 06.12.2005
(51) Int. Cl.: C07D 491/04

(54) **UTILISATION DE LA CHROMATOGRAPHIE DE PARTAGE CENTRIFUGE POUR LA PURIFICATION DE LA GALANTHAMINE**
ANWENDUNG VON ZENTRIFUGALER VERTEILUNGSCHROMATOGRAPHIE ZUR AUFREINIGUNG VON GALANTHAMINE
USE OF CENTRIFUGAL PARTITION CHROMATOGRAPHY FOR PURIFYING GALANTHAMINE

(30) Priorité: 16.12.2004 FR 0413425
(43) Date de publication de la demande: 29.08.2007
(73) Titulaire: Centre National de la Recherche Scientifique, 75794 Paris Cedex 16 (FR); Université De Reims Champagne-Ardenne, 51100 Reims (FR)
(72) Inventeur: RENAULT, Jean-Hugues, 51100 Reims (FR); NUZILLARD, Jean-Marc, 51390 Courmas (FR); MACIUK, Alexandre, 68000 Colmar (FR); ZECHES-HANROT, Monique, 51430 Bezannes (FR)
(74) Mandataire: Grosset-Fournier, Chantal Catherine
(86) Numéro de dépôt international: PCT/FR2005/003046
(87) Numéro de publication internationale: WO 2006/064105

(56) Documents cités:
- WO-A-00/58722
- US-A1- 2002 028 802
- US-B1- 6 346 618
- US-B1- 6 407 229
- DATABASE WPI Section Ch, Week 200447 Derwent Publications Ltd., London, GB; Class E13, AN 2004-488415 XP002336902 & CN 1 490 319 A (YIXIN PHARM CO LTD ZHEJIANG PROV) 21 avril 2004 (2004-04-21)

## Description

La présente invention a pour objet l'utilisation de la chromatographie de partage centrifuge pour la purification de la galanthamine.

La chromatographie de partage centrifuge (CPC) est une technique de chromatographie liquide-liquide sans support solide (Foucault, 1994 ; Berthod, 2002), qui requiert l'utilisation d'un système biphasique élaboré à partir d'au moins deux solvants et/ou solutions. Les colonnes utilisées sont de deux types : soit elles sont constituées d'un cylindre creux dont la paroi est creusée de cellules de partage distribuées radialement et reliées entre elles par des conduits de section inférieure à celle des cellules de partage (voir demande de brevet français 2,802,104 ou demande internationale WO 2004/079363 au nom de Partus Technologies) ; soit elles sont constituées de disques empilés gravés de cellules de partage reliées entre elles par des conduits de section inférieure où l'étanchéité entre les disques est assurée par des joints en Téflon^{®} de même diamètre que les disques (demande internationale WO 00/58722 au nom de Kromaton Technologies). Le champ de force centrifuge constant généré, par la rotation de la colonne maintient une des phases stationnaire dans cette dernière, l'autre phase étant pompée au travers et jouant le rôle de phase mobile. La réalisation d'une séparation par CPC est principalement caractérisée par la sélection d'un système de solvants adapté à une séparation particulière (Renault et al., 2002).

Un autre élément essentiel d'une séparation par CPC est le choix du mode de développement chromatographique. L'élution est un mode commun à la chromatographie liquide haute performance (CLHP) et à la CPC, où les analytes ne sont soumis qu'aux lois thermodynamiques des équilibres de partage entre solvants. Ce mode se caractérise idéalement par l'élution des analytes selon un profil de concentration gaussien. Un autre mode, appelé déplacement, et décrit pour la première fois par Tiselius (Tiselius, 1943), est fondé sur la compétition des analytes entre eux lors de la chromatographie. Deux conditions doivent être remplies pour que ce phénomène soit observé : le comportement non linéaire de chacun des analytes et la phase mobile ne peut pas mobiliser seule les analytes. Il faut pour cela introduire en solution dans la phase mobile un déplaceur qui présente une interaction avec la phase stationnaire plus forte que tous les analytes. Un exemple est constitué par la chromatographie d'échange d'ions en CPC ou sur support solide (Maciuk et al., 2004). La phase stationnaire contient alors un échangeur (cationique ou anionique, fort ou faible) et les analytes progressent dans la colonne sous l'action du déplaceur. Les composés s'organisent dans la colonne en une succession de zones dans lesquelles les concentrations sont constantes, l'ordre d'émergence des composés étant fixé par leurs constantes d'association avec l'échangeur. Ce train de composés, appelé train isotachique, progresse dans la colonne à une vélocité fonction du rapport stoechiométrique entre la concentration en reteneur (composé destiné à maintenir l'analyte dans la phase stationnaire) et la concentration en déplaceur (Maciuk et al., 2004 ; Intes et al., 2001). Les profils de concentration des espèces émergentes sont alors rectangulaires et présentent des zones de transition abruptes appelées "schock layers". Ce dernier mode est particulièrement adapté aux applications préparatives. Une variante propre à la chromatographie liquide-liquide sans support solide similaire dans son principe au "pH-zone refining" revendiqué en Chromatographie à Contre-Courant (chromatographie liquide-liquide sans support solide en mode hydrodynamique)(brevets américains US 5,332,504, US 5,354,473 et US 5,449,461 ; Weisz et al., 1994) est obtenue en mettant à profit des équilibres acido-basiques entre les analytes. Les composés séparables par ce mode chromatographique doivent être ionisables et les formes neutres et ionisées doivent présenter des différences de polarité suffisante pour inverser leurs constantes de distributions dans le système biphasique sélectionné (Ito et al., 1996 ; Ito et al., 1995 ; Renault et al., 1999).

Outre les avantages connus et fréquemment cités de la CPC tels que l'absence d'adsorption irréversible des solvants, la consommation réduite de solvants, l'obtention de sélectivités élevées, il apparaît que cette technique possède d'indéniables qualités préparatives (Margraff et al., 1994 ; Renault et al., 1999).

La (-)-galanthamine naturelle (C₁₇H₂₁NO₃ ; M= 287,2) est une amine répondant à la formule suivante :

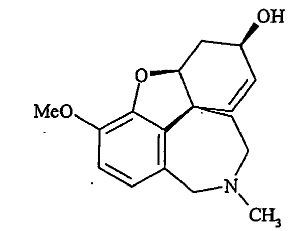

La (+)-galanthamine, accessible par synthèse, est l'énantiomère 12a(*R*),4a(*R*),3(*S*) de la galanthamine, et n'a pas d'activité biologique.

La(-)-galanthamine est un inhibiteur compétitif de l'acétylcholinestérase dans le traitement des symptômes de la maladie d'Alzheimer. Les traitements disponibles à l'heure actuelle sont seulement symptomatiques et se basent tous sur l'hypothèse cholinergique de la maladie. Parmi les molécules sur le marché actuellement, la galanthamine est la seule substance naturelle, présente dans les bulbes et/ou les parties aériennes de certaines plantes de la famille des Amaryllidaceae. Elle est notamment commercialisée par les laboratoires Janssen-Cilag (Réminyl^{®} qui est un sel bromhydrate de (-)-galanthamine), mais sa production par Sanochemia. Pharmazeutica AG (Vienne, Autriche) est assurée par la voie de la synthèse chimique, notamment selon les procédés décrits dans la demande internationale WO 97/45431 ou dans le brevet américain US 6,407,229.

Dans le cadre de ces procédés, l'obtention de galanthamine optiquement pure implique l'utilisation de réactifs chiraux et/ou d'une cristallisation fractionnée à partir d'un sel d'acide organique optiquement pur (acide tartrique). Il est également parfois nécessaire d'avoir recours à de la chromatographie sur support solide (gel de silice). De tels procédés sont donc longs et relativement coûteux à mettre en oeuvre.

Par ailleurs, les brevets américains US 6,573,376, US 6,617,452 et US 6,194,404 concernent un procédé d'isolement de la galanthamine, comprenant une étape d'extraction et une étape de purification par recristallisation. Les procédés de cristallisation de l'art antérieur, notamment mis au point pour un extrait de *Narcissus pseudonarcissus* "Carlton", nécessitent a priori une extraction très sélective afin d'enrichir au maximum l'extrait en galanthamine. De plus, de tels procédés peuvent s'avérer inefficaces dans le cas d'un extrait obtenu à partir d'une plante différente. Enfin, le rendement d'extraction est particulièrement faible (0,01% à partir des bulbes secs) à partir d'une drogue titrant entre 0,2 et 0,3%.

Ces procédés de purification sont des procédés lourds et difficiles à mettre en oeuvre et présentent un coût élevé.

Ces procédés de purification sont limités par l'utilisation de supports chromatographiques solides, ce qui affecte les rendements en produits isolés, et par l'utilisation de quantités importantes de solvants par rapport aux masses produites,

Le brevet CN 1 490 319 concerne un procédé d'isolement de la galanthamine par CCC.

La présente invention a pour but de fournir un procédé de purification de la galanthamine ou de ses dérivés, conduisant à l'obtention de composés dans un état de pureté compatible avec les exigences de l'industrie pharmaceutique, et avec des coûts de production inférieurs à ceux obtenus lors de l'utilisation de techniques de chromatographie sur support solide.

La présente invention a pour but de fournir un procédé de purification de la galanthamine reproductible et d'industrialisation aisée.

La présente invention concerne l'utilisation d'un procédé de chromatographie de partage centrifuge en mode déplacement, pour la mise en oeuvre d'un procédé de purification de la galanthamine ou de ses dérivés.

L'expression "mode déplacement" correspond à un mode de CPC différent du mode d'élution. Ce mode est proche dans son principe de l'échange d'ions.

L'expression "galanthamine ou ses dérivés" désigne la galanthamine ou les dérivés de la galanthamine tels que : la sanguinine, la galanthaminone (ou narwedine), la galanthaminone, la norgalanthamine, la 11-hydroxygalanthamine, lycoramine, lycorine, assoanine, etc.

Le composé purifié obtenu, correspondant à la galanthamine ou à l'un de ses dérivés, présente une pureté supérieure à environ 99%, et avantageusement supérieure à environ 99,2%.

Le procédé de l'invention permet d'accéder à des sélectivités chromatographiques très élevées et à des rendements de purification dépassant 90%. De plus, l'utilisation de la CPC permet de réduire la quantité de solvant d'un facteur compris entre 5 et 10 comparativement à la chromatographie liquide CLHP, généralement utilisée pour ce type de purification. Cette économie de solvant est directement imputable à la forte proportion de phase stationnaire entièrement accessible dans la colonne (classiquement entre 30 et 80% du volume total de la colonne), ce qui limite la dilution des analytes. De plus, il faut souligner que l'absence de phase solide permet une reproductibilité élevée en évitant les problèmes de compactage progressif du support chromatographique. Enfin, comparativement à la cristallisation, le procédé est plus adaptable aux différentes sources de galanthamine potentielles (plantes différentes, synthèse, biotechnologies), et donne de bien meilleurs rendements, en moyenne supérieurs à 90%.

Le procédé de l'invention permet une flexibilité, dans la mesure où le composé purifié peut être obtenu sous la forme d'une base ou d'un sel, selon l'utilisation ultérieure souhaitée.

Dans le cadre de l'utilisation de la galanthamine dans des médicaments, la galanthamine est sous forme bromhydrate. Ainsi, lorsque le composé est obtenu sous la forme d'une base, il est nécessaire de le transformer en bromhydrate et cette étape supplémentaire permet d'améliorer la pureté de la galanthamine car elle permet la perte d'impuretés supplémentaires.

La présente invention concerne l'utilisation telle que définie ci-dessus, à partir d'une composition de départ, contenant au moins 20% de galanthamine ou de ses dérivés, ledit procédé comprenant une étape de centrifugation d'une combinaison d'au moins deux solvants et de ladite composition de départ, pendant un temps suffisant pour purifier la galanthamine ou ses dérivés,
lesdits solvants étant tels qu'ils forment deux phases non miscibles, à savoir une phase aqueuse et une phase organique, la phase aqueuse servant de phase mobile ou de phase stationnaire, et la phase organique servant respectivement de phase stationnaire ou de phase mobile.

Le procédé de la présente invention comprend une étape dans laquelle on centrifuge d'une part la composition de départ telle que définie ci-dessus et d'autre part une combinaison d'au moins deux solvants, telle que définie ci-dessus.

La combinaison de solvants comprend toujours au moins deux solvants, dans la mesure où le procédé de l'invention nécessite la présence de deux phases, à savoir une phase organique et une phase aqueuse, ce qui n'est pas possible en cas d'utilisation d'un solvant unique.

Dans le cadre de l'étape de centrifugation, le champ de force centrifuge créé permet de maintenir une phase stationnaire dans la colonne. De manière générale, l'opérateur aura intérêt à générer une rotation la plus élevée possible car cela améliore le transfert de matière en augmentant la surface d'échange entre la phase mobile et la phase stationnaire. La limite supérieure est fonction de la tolérance de l'appareillage à la perte de charge (cette dernière augmente avec la rotation ; le maximum est de 60 bars sur les appareils de laboratoire classiques et de 150 bars sur l'appareil industriel commercialisé par Partus). Classiquement, le champ de force appliqué est au minimum de 100 g.

L'expression "pendant un temps suffisant pour purifier la galanthamine ou ses dérivés" correspond à une durée d'environ 20 minutes à plusieurs heures.

L'expression "composition de départ" désigne une composition contenant au moins le composé à purifier, à savoir la galanthamine ou l'un de ses dérivés, c'est-à-dire contenant au moins un composé alcaloïdique. Cette composition de départ correspond à la composition soumise au procédé de purification, avant l'étape de centrifugation.

Selon un mode de réalisation avantageux de la présente invention, la composition de départ contient le composé à purifier, sous forme de sel ou d'amine, dissous dans la phase stationnaire ou la phase mobile, ladite composition de départ étant saturée ou non respectivement par l'ajout de phase mobile ou de phase stationnaire.

Selon un mode de réalisation avantageux, le composé à purifier est récupéré danse la phase organique, dans la mesure où il sera plus facile à récupérer dans cette phase susceptible de s'évaporer plus facilement que la phase aqueuse.

Dans le cadre du procédé de l'invention, lorsque la phase stationnaire est la phase aqueuse, la phase mobile est la phase organique. Inversement, lorsque la phase stationnaire est la phase organique, la phase mobile est la phase aqueuse.

La présente invention concerne un procédé de purification de la galanthamine ou de ses dérivés à partir d'une composition de départ, contenant au moins 20% de galanthamine ou de ses dérivés, par chromatographie de partage centrifuge en mode déplacement, ledit procédé comprenant une étape de centrifugation
- d'une combinaison d'au moins deux solvants, et de préférence d'au moins trois solvants, et
- de ladite composition de départ
pendant un temps suffisant pour purifier la galanthamine ou ses dérivés,
lesdits solvants étant tels qu'ils forment deux phases non miscibles, à savoir une phase aqueuse et une phase organique, la phase aqueuse servant de phase mobile ou de phase stationnaire, et la phase organique servant respectivement de phase stationnaire ou de phase mobile.

Un procédé avantageux selon la présente invention est **caractérisé en ce que** la composition de départ est un extrait de plante ou de la matière biologique produisant de la galanthamine ou ses dérivés, telle que des cellules de plantes, ou un mélange de composés obtenu par synthèse organique contenant notamment de la galanthamine et/ou ses dérivés.

Les composés obtenus par synthèse organique nécessitent également une étape de purification, qui peut être dans certains cas une étape de chromatographie. De plus, si on sépare par le procédé de l'invention un mélange racémique, on obtient le racémate purifié des autres impuretés.

Afin d'être introduit en tête de colonne, ledit extrait de plante ou ladite matière biologique ou ledit mélange est dissous dans la phase mobile ou la phase stationnaire.

Le choix pour la phase qui servira à dissoudre la composition de départ est guidé par deux critères :
- la solubilité : on choisira de solubiliser la composition de départ dans la phase où elle présente la meilleure solubilité pour des raisons évidentes de productivité,
- l'influence de la composition de départ sur l'équilibre du système biphasique : il arrive que la dissolution dans une phase conduise à une déstabilisation moindre du système biphasique, ce qui conduira à un meilleur profil d'injection et donc à une meilleure rétention de phase stationnaire et donc une meilleure séparation (Marchal et al., 2003).

Les composés obtenus par synthèse organique nécessitent également une étape de purification, qui peut être dans certains cas une étape de chromatographie.

Si on sépare par le procédé de l'invention un mélange racémique, on obtient le racémate purifié des autres impuretés.

Un procédé particulièrement avantageux selon la présente invention est **caractérisé en ce que** les deux phases liquides non miscibles correspondent à une combinaison d'au moins deux solvants, à savoir de l'eau et un solvant non miscible ou partiellement miscible à l'eau, et forment ainsi une phase aqueuse et une phase organique.

L'expression "solvant partiellement miscible à l'eau" désigne un solvant qui n'est pas totalement miscible à l'eau.

Ainsi, par exemple, le système butanol/eau est un système biphasique dans lequel une importante quantité de butanol est présente dans la phase aqueuse et dans lequel une importante quantité d'eau est présente dans la phase organique.

La phase aqueuse correspond à une phase riche en eau, c'est-à-dire essentiellement constituée d'eau. Elle contient également une petite quantité de solvant(s) non miscible(s) ou partiellement miscible(s) à l'eau.

La phase organique correspond à une phase riche en solvant(s) partiellement ou non miscible(s) à l'eau, c'est-à-dire essentiellement constituée de solvant(s) non miscible(s) ou partiellement miscible(s) à l'eau. Elle contient également une petite quantité d'eau.

La présente invention concerne un procédé tel que défini ci-dessus, **caractérisé en ce que** les deux phases liquides non miscibles correspondent à un mélange d'au moins trois solvants, à savoir de l'eau, un solvant non miscible ou partiellement miscible à l'eau et un "solvant pont", ledit solvant pont étant un solvant partiellement ou totalement soluble dans l'eau et dans le solvant non miscible ou partiellement miscible à l'eau,
lesdits solvants étant tels qu'ils forment un système biphasique comprenant une phase aqueuse et une phase organique.

L'expression "solvant pont" est un terme utilisé dans le cadre de la présente invention afin de désigner un solvant à la fois miscible dans la phase organique et dans la phase aqueuse. Il est donc présent à la fois dans les deux phases.

L'expression "solvant partiellement soluble dans l'eau et dans le solvant non miscible ou partiellement miscible à l'eau" désigne un solvant présent à la fois dans la phase organique et dans la phase aqueuse.

L'expression "système biphasique" désigne un système présentant deux phases, qui sont toutes les deux liquides dans le cas de la présente invention.

Selon un mode de réalisation avantageux de la présente invention, le procédé tel que défini ci-dessus est **caractérisé en ce que** le solvant non miscible ou partiellement miscible à l'eau est choisi parmi :
- les éthers tels que l'éther de méthyle et de tertiobutyle (MTBE) et l'éther d'éthyle et de tertiobutyle,
- les cétones non miscibles à l'eau telles que la méthyléthylcétone (MEK) et la méthylisobutylcétone (MIBK),
- les hydrocarbures aromatiques tels que le toluène,
- les hydrocarbures aliphatiques tels que l'hexane, l'heptane et les cyclohexanes,
- les éthers de pétrole,
- les alcools lourds dont la chaîne carbonée comprend au moins 4 atomes de carbone, tels que le n-butanol, le 2-butanol et l'isobutanol,
- les siloxanes non miscibles à l'eau tels que l'hexaméthyldisiloxane,
- les solvants halogénés non miscibles à l'eau, tels que le chloroforme, le dichlorométhane et le dichloro-1,2-éthane, ou
- les esters tels que l'acétate d'éthyle et l'acétate de butyle.

Parmi tous ces solvants, les solvants halogénés sont les seuls à être plus lourds que l'eau.

Les familles susmentionnées de solvants sont des familles de solvants non- ou partiellement miscibles à l'eau. Ces solvants génèrent donc une phase moins polaire que la phase aqueuse. Cette phase organique présente donc des propriétés physico-chimiques telles qu'une constante diélectrique et un moment dipolaire inférieur à l'eau (recouvrant la notion de solvant apolaire) susceptible de permettre la dissolution préférentielle des alcaloïdes (forme basique).

La présente invention concerne un procédé tel que défini ci-dessus, **caractérisé en ce que** le solvant "pont" est choisi parmi : les alcools légers dont la chaîne carbonée comprend moins de 4 atomes de carbone tels que le méthanol, l'éthanol, les propanols, l'acétonitrile, l'acétone, le tétrahydrofuranne, le diméthylsulfoxyde et le diméthylformamide.

Ces solvants sont de "bons solvants" des alcaloïdes (forme sel et forme base) facilitant la solubilisation générale et également le transfert de matière entre les deux phases.

La présente invention concerne un procédé tel que défini ci-dessus, **caractérisé en ce que** la phase mobile contient un déplaceur qui est un acide ou une base.

En mode déplacement en général, les solvants constituant la phase mobile ne sont pas éluants à eux seuls. Ils sont incapables de mobiliser les composés à purifier présents dans la composition de départ.

Une espèce supplémentaire, introduite dans le système par la phase mobile, déplace les composés à purifier présents dans la composition de départ de leur fixation sur la phase stationnaire : c'est le déplaceur, ou développeur. Tout comme le reteneur, il présente le même type d'interaction avec la phase stationnaire que les composés à purifier, mais avec une constante d'association avec l'échangeur plus grande que ces derniers. C'est le déplaceur qui est à l'origine de la force motrice de la chromatographie.

En mode "pH-zone-refining", le déplaceur est une espèce présente dans la phase mobile, qui force le composé à purifier à passer dans la phase mobile, en faisant changer son état d'ionisation par des réactions acido-basiques. Dans le cas d'une séparation d'alcaloïdes, si la phase mobile est organique, le déplaceur doit être une base forte liposoluble capable de neutraliser les composés à purifier et de diminuer leur K_{D} (constante de distribution). Si la phase mobile est aqueuse, le déplaceur doit être un acide fort hydrosoluble capable de protonner les composés à purifier et d'augmenter leur K_{D}. Le déplaceur est nécessairement ajouté à la phase mobile, il ne peut pas être injecté avec l'échantillon. C'est sa concentration qui détermine la vélocité des composés à purifier dans la colonne.

Selon la présente invention, le déplaceur est un composé acido-basique présent dans la phase mobile aqueuse ou organique.

Un procédé avantageux selon la présente invention est un procédé tel que défini ci-dessus, **caractérisé en ce que** la phase stationnaire correspond à la phase aqueuse et la phase mobile correspond respectivement à la phase organique, et en ce que le déplaceur contenu dans la phase mobile organique est une base.

Selon ce mode de réalisation, le composé alcaloïdique purifié, à savoir la galanthamine ou l'un de ses dérivés, est élué dans la phase mobile, c'est-à-dire dans la phase organique ; il est donc sous sa forme basique, c'est-à-dire sous une forme amine. Ainsi, le composé alcaloïdique à purifier est initialement sous sa forme acide, à savoir sous la forme d'un sel d'ammonium.

Un procédé avantageux selon la présente invention est un procédé tel que défini ci-dessus, **caractérisé en ce que** la phase stationnaire correspond à la phase organique et la phase mobile correspond respectivement à la phase aqueuse, et en ce que le déplaceur contenu dans la phase mobile aqueuse est un acide.

Selon ce mode de réalisation, le composé purifié, à savoir la galanthamine ou l'un de ses dérivés, est élué dans la phase mobile, c'est-à-dire dans la phase aqueuse ; il est donc sous sa forme acide, c'est-à-dire sous sa forme de sel d'ammonium. Ainsi, la forme initiale du composé à purifier est sa forme basique, à savoir la forme amine.

Un procédé préféré selon l'invention est **caractérisé en ce qu**'un reteneur est :
- soit ajouté dans la composition de départ ou dans la phase stationnaire,
- soit est un élément de la composition de départ,
ledit reteneur étant un acide ou une base.

L'expression "reteneur" désigne un composé soumis au processus de purification ayant la plus grande différence de pKa avec le déplaceur tel que défini ci-dessus.

On donne le nom de reteneur à une espèce de même caractère acido-basique que le composé à purifier, mais montrant une plus grande différence de pKa avec le déplaceur que toutes les espèces présentes. Le reteneur maintient le composé à purifier dans la phase stationnaire. Le reteneur peut être présent dans la phase stationnaire, soit être injecté avec l'échantillon (un cas particulier est alors de considérer que le composé de la composition de départ présentant la plus grande différence de pKa avec le déplaceur joue ce rôle). Dans le cas d'un composé à purifier basique, si la phase stationnaire est aqueuse, le reteneur doit être un acide fort hydrosoluble, capable d'ioniser le composé à purifier afin de favoriser son partage dans la phase stationnaire (en augmentant sa constante de distribution K_{D} qui doit être >> 1). Si la phase stationnaire est organique, le reteneur doit être une base forte liposoluble capable de neutraliser le composé à purifier et d'augmenter sa constante de distribution qui doit être << 1).

Le procédé de l'invention permet donc de contrôler le couple pureté / temps de purification, dans la mesure où les quantités de déplaceur et de reteneur - régissant la vitesse de progression du train isotachique de composés - peuvent être modulées de façon à obtenir un temps d'émergence des composés juste suffisant pour obtenir la pureté désirée.

Selon un mode de réalisation préféré, le procédé de l'invention est **caractérisé en ce qu**'on ajoute dans la composition de départ ou dans la phase stationnaire un reteneur, qui est un acide.

Selon ce mode de réalisation, la phase mobile organique contient un déplaceur basique et la phase stationnaire est la phase aqueuse, dans laquelle on ajoute un reteneur acide possédant une constante d'acidité plus forte que celles des formes protonées des composés alcaloïdiques à purifier.

Selon un mode de réalisation préféré, le procédé de l'invention est **caractérisé en ce qu**'on ajoute dans la composition de départ ou dans la phase stationnaire un reteneur, qui est une base.

Selon ce mode de réalisation, la phase mobile aqueuse contient un déplaceur acide et la phase stationnaire est la phase organique, dans laquelle on ajoute un reteneur basique possédant une constante d'acidité plus faible que celles des composés alcaloïdiques à purifier.

Selon un mode de réalisation avantageux, le procédé de la présente invention est **caractérisé en ce que** la composition de départ contient les alcaloïdes sous forme de sels d'ammonium dissous dans la phase stationnaire aqueuse, la composition de départ étant alors saturée ou non par un ajout de phase mobile organique sans déplaceur.

Un des intérêts de la CPC est de pouvoir injecter en phase mobile ou en phase stationnaire. Trois aspects sont à considérer :
- la solubilité de la composition de départ (il faut en dissoudre le plus possible pour être productif...),
- l'influence de la composition de départ sur le caractère biphasique du système de solvant, et dans ce cas une des deux phases peut présenter une meilleure affinité que l'autre (Marchal et al., 2003),
- la qualité finale de la séparation.

Il a été montré qu'une des conditions de réussite d'une séparation était le contrôle du caractère saturé en phase conjuguée (phase organique ou phase aqueuse) de la phase dans laquelle l'échantillon a été dissous (respectivement phase aqueuse ou phase organique)(Marchal et al., 2003). C'est pourquoi une quantité de phase conjuguée "vierge" (phase organique ou phase aqueuse) peut-être ajoutée afin de resaturer le volume d'injection.

Selon un autre mode de réalisation avantageux, le procédé de la présente invention est **caractérisé en ce que** la composition de départ contient les alcaloïdes sous forme de bases neutres dissoutes dans la phase mobile aqueuse, la composition de départ étant alors saturée ou non par un ajout de phase stationnaire organique sans reteneur.

Selon un autre mode de réalisation avantageux, le procédé de la présente invention est **caractérisé en ce que** la composition de départ contient les alcaloïdes sous forme de bases neutres dissoutes dans la phase stationnaire organique, la composition de départ étant alors saturée ou non par un ajout de phase mobile aqueuse sans déplaceur.

Selon un autre mode de réalisation avantageux, le procédé de la présente invention est **caractérisé en ce que** la composition de départ contient les alcaloïdes sous forme de sels d'ammonium dissous dans la phase mobile organique, la composition de départ étant alors saturée ou non par un ajout de phase stationnaire aqueuse sans reteneur.

La présente invention concerne également un procédé tel que défini ci-dessus, comprenant les étapes suivante :
- l'injection de la phase stationnaire dans une colonne de chromatographie de partage centrifuge, ladite phase stationnaire contenant un reteneur tel que défini ci-dessus, afin d'obtenir une colonne de chromatographie de partage centrifuge remplie de phase stationnaire,
- l'injection de la composition de départ telle que définie ci-dessus dans la colonne de chromatographie de partage centrifuge remplie de phase stationnaire, pour obtenir une colonne de chromatographie de partage centrifuge chargée avec ladite phase stationnaire et ladite composition de départ, et
- l'introduction par pompage dans la colonne telle qu'obtenue à l'étape précédente, de la phase mobile dans laquelle un déplaceur est ajouté telle que définie ci-dessus, afin d'éluer la galanthamine ou ses dérivés.

L'expression "colonne de chromatographie de partage centrifuge remplie de phase stationnaire" désigne une colonne, correspondant à une succession de cavités parallélépipédiques ou cylindriques, reliées entre elles par des canaux. Au départ, la colonne est vide. Chaque séparation par CPC commence donc par le remplissage total de la colonne, en général en appliquant une rotation faible (100 à 300 rpm).

Une fois la colonne remplie de phase stationnaire, la rotation de travail est appliquée (classiquement entre 100 et 500 g). L'échantillon est injecté via une boucle d'injection ou via la pompe en étant poussé par la phase mobile. La phase mobile va déplacer un volume de phase stationnaire dans chaque cellule de partage qui dépend du système de solvants, de la rotation, du débit, jusqu'à l'atteinte d'un équilibre hydrodynamique. Cet équilibre se caractérise par le rapport volume de phase stationnaire/volume total de la colonne (noté Sf et appelé rétention). Concrètement, au début de l'expérience, on observe la sortie de phase stationnaire, jusqu'à atteinte de l'équilibre, détecté par l'apparition (la sortie) de phase mobile. A cet état d'équilibre, à chaque volume de phase mobile pompé il sort le même volume de phase mobile de la colonne. Un système chromatographique en CPC est donc en partie caractérisé par le taux de rétention de phase stationnaire dans la colonne. Classiquement, des séparations sont possibles avec des rétentions comprises entre 30 et 85%.

Le procédé de l'invention tel que défini ci-dessus est **caractérisé en ce que** le déplaceur est choisi parmi :
- les acides minéraux tels que HCl ou H₂SO₄,
- les acides organiques tels que l'acide méthane-sulfonique, l'acide trifluoroacétique, l'acide acétique, l'acide tartrique ou l'acide citrique,
- les bases minérales telles que l'ammoniac ou la soude, ou
- les bases organiques telles que la triéthylamine.

Lorsque le déplaceur est un acide, il est plus acide que les acides conjugués des composés présents dans la composition de départ, et lorsque le déplaceur est une base, il est plus basique que les composés présents dans la composition de départ.

Les acides minéraux sont les produits les plus couramment utilisés en tant que déplaceurs. Dans le cas particulier de la galanthamine, HBr peut être intéressant dans la mesure où la forme pharmaceutique est le bromhydrate de galanthamine. L'acide tartrique est intéressant car il est alors possible de sortir les alcaloïdes sous forme de tartrates qui peuvent être des sels pharmaceutiques.

Parmi les bases, les déplaceurs préférés sont les bases organiques car dans ce cas, le déplaceur basique doit montrer une solubilité correcte dans la phase organique.

Le procédé de l'invention tel que défini ci-dessus est **caractérisé en ce que** le reteneur est choisi parmi :
- les bases minérales telles que l'ammoniac ou la soude, ou
- les bases organiques telles que la triéthylamine.
- les acides minéraux tels que HCl ou H₂SO₄,
- les acides organiques tels que l'acide méthane-sulfonique, l'acide, trifluoroacétique, l'acide acétique, l'acide tartrique ou l'acide citrique.

Lorsque le reteneur est un acide, il est plus acide que les acides conjugués des composés présents dans la composition de départ et, lorsque le reteneur est une base, il est plus basique que les composés présents dans la composition de départ.

Selon un mode de réalisation avantageux de l'invention, le procédé de l'invention comprend l'utilisation d'une combinaison des solvants suivants :
- toluène, heptane, acétone et eau, ou
- éther de méthyle et de tertiobutyle, acétonitrile et eau, ou
- éther de méthyle et de tertiobutyle, acétone et eau, ou
- méthylisobutylcétone, acétone et eau.

La présente invention concerne également un procédé tel que défini ci-dessus, comprenant l'utilisation de la combinaison suivante de solvants : toluène, heptane, acétone et eau, dans des proportions volumiques telles que :
- le pourcentage volumique du toluène est supérieur à celui de l'heptane,
- le pourcentage volumique de l'acétonitrile ne dépasse pas 50%, et
- le mélange de ces solvants est biphasique,
et notamment dans les proportions volumiques 24:8:10:34.

La présente invention concerne également un procédé tel que défini ci-dessus, comprenant l'utilisation de la combinaison suivante de solvants : éther de méthyle et de tertiobutyle, acétonitrile et eau, dans des proportions volumiques telles que :
- le pourcentage volumique de l'acétonitrile ne dépasse pas 45%, et
- le mélange de ces solvants est biphasique,
et notamment dans les proportions volumiques 4:1:5.

Selon un mode de réalisation avantageux, le procédé de l'invention est **caractérisé en ce que** la composition de départ est un extrait de parties aériennes ou de bulbes d'une Amaryllidaceae, notamment des genres *Leucojum, Narcissus* ou *Galanthus,* et est de préférence un extrait de feuilles de *Leucojum aestivum* ou un extrait de bulbes de *Narcissus carlton.*

Selon un mode de réalisation avantageux, le procédé de l'invention est **caractérisé en ce que** la composition de départ est un extrait de feuilles de *Leucojurn aestivum* et en ce que la combinaison de solvants est la suivante : toluène, heptane, acétone et eau, dans les proportions volumiques 24:8:10:34.

Selon un mode de réalisation avantageux, le procédé de l'invention est **caractérisé en ce que** la composition de départ est un extrait de bulbes de *Narcissus carlton* et en ce que la combinaison de solvants est la suivante : éther de méthyle et de tertiobutyle, acétonitrile et eau, dans les proportions volumiques 4:1:5.

Le procédé de la présente invention peut être effectué en mode ascendant ou en mode descendant. Le mode ascendant correspond au cas où la phase mobile est plus légère que la phase stationnaire, tandis que le mode descendant correspond au cas où la phase mobile est plus lourde que la phase stationnaire.

Dans le cadre de la présente invention, la phase organique est toujours plus légère que la phase aqueuse, sauf lorsque la phase organique comprend des solvants chlorés, tels que le chloroforme, le dichlorométhane et le dichloro-1,2-éthane.

La présente invention concerne également un procédé tel que défini ci-dessus, **caractérisé en ce qu**'il comprend les étapes suivantes :
- l'injection de la phase stationnaire aqueuse dans une colonne de chromatographie de partage centrifuge, ladite phase stationnaire contenant un reteneur acide tel que défini ci-dessus, afin d'obtenir une colonne de chromatographie de partage centrifuge remplie de phase stationnaire acidifiée,
- l'injection de la composition de départ, dans laquelle la galanthamine ou ses dérivés sont sous forme de sels, dans la colonne de chromatographie de partage centrifuge remplie de phase stationnaire acidifiée, pour obtenir une colonne de chromatographie de partage centrifuge chargée avec ladite phase stationnaire acidifiée et ladite composition de départ, et
- l'introduction par pompage de la phase mobile organique à travers la colonne telle qu'obtenue à l'étape précédente, dans laquelle un déplaceur basique est ajouté, afin d'éluer la galanthamine ou ses dérivés sous forme basique.

Dans le cadre de la mise en oeuvre du procédé susmentionné, juste avant l'injection de la phase mobile organique, la colonne contient en tête, dans les premières cellules de partage, l'échantillon (analyte sous forme de sel) puis la phase stationnaire aqueuse dans laquelle le reteneur acide a été ajouté. Ensuite, on injecte la phase mobile organique contenant le déplaceur basique. Le déplaceur basique prend alors le proton du sel de l'analyte le moins basique de la composition de départ et entraîne cet analyte (forme amine) dans la phase mobile organique, qui avance dans la colonne en raison du pompage. Ainsi, l'analyte (forme amine) déplacé est protoné par le reteneur acide lors du contact avec la phase stationnaire et se retrouve alors sous sa forme de sel dans la phase stationnaire.

Le procédé de l'invention correspond donc à un enchaînement de différenties réactions acido-basiques qui entraînent le déplacement du composé à purifier entre la phase stationnaire et la phase aqueuse.

Selon un mode de réalisation avantageux, le procédé tel que défini ci-dessus, notamment dans le paragraphe ci-dessus, est **caractérisé en ce que** le reteneur acide est ajouté dans la phase stationnaire.

La présente invention concerne également un procédé tel que défini ci-dessus, **caractérisé en ce qu**'il comprend les étapes suivantes :
- l'injection de la phase stationnaire organique dans une colonne de chromatographie de partage centrifuge, ladite phase stationnaire contenant un reteneur basique tel que défini ci-dessus, afin d'obtenir une colonne de chromatographie de partage centrifuge remplie de phase stationnaire alcalinisée,
- l'injection de la composition de départ, dans laquelle la galanthamine ou ses dérivés sont sous forme basique, dans la colonne de chromatographie de partage centrifuge remplie de phase stationnaire alcalinisée, pour obtenir une colonne de chromatographie de partage centrifuge chargée avec ladite phase stationnaire alcalinisée et ladite composition de départ, et
- l'introduction par pompage de la phase mobile aqueuse à travers la colonne telle qu'obtenue à l'étape précédente, dans laquelle un déplaceur acide est ajouté, afin d'éluer la galanthamine ou ses dérivés sous forme de sels.

Selon un mode de réalisation avantageux, le procédé tel que défini ci-dessus, notamment dans le paragraphe ci-dessus, est **caractérisé en ce que** le reteneur basique est ajouté dans la phase stationnaire.

### PARTIE EXPÉRIMENTALE

### EXEMPLE 1 - Mode ascendant Leucojum aestivum

### 1- Préparation de l'extrait :

1,660 kg de feuilles de *Leucojum aestivum* broyées (grille 2mm) sont humectés par 1 litre d'ammoniaque 10% dans l'eau. Le mélange est placé dans un percolateur puis est mis à macérer dans 30 1 d'acétate d'éthyle pendant 18h. Ensuite, l'acétate d'éthyle est lixivié jusqu'à épuisement. La solution extractive est extraite par 3 × 3 l puis 3 × 2l d'eau sulfurique à 3%. La phase aqueuse est alcalinisée à pH environ 10 par de l'ammoniaque à 20% dans l'eau. Cette solution aqueuse est extraite par 2 × 3l puis 1 × 2 l de chloroforme. La solution chloroformique est lavée à l'eau jusqu'à obtenir un pH d'environ 7, séchée sur sulfate de sodium puis évaporée à siccité sous pression réduite pour obtenir 3,639g d'extrait d'alcaloïdes totaux, soit un rendement de 2,192g/kg de feuilles sèches.

### 2- Séparation par Chromatographie de Partage Centrifuge, en mode déplacement :

### a- Appareillage :

L'appareil utilisé est un Chromatographe de Partage Centrifuge FCPC Kromaton^{®}, d'une capacité de 200 ml (Kromaton Technologies, Angers, France).

### b- Système biphasique de solvants :

Le système utilisé correspond au mélange toluène/heptane/acétone/eau 24:8:10:34 (v/v). Les solvants sont agités dans une ampoule à décanter, puis séparés. La phase aqueuse est acidifiée par de l'acide méthane-sulfonique (reteneur) à une concentration de 10 mM. La phase organique est alcalinisée par de la triéthylamine (déplaceur) à une concentration de 8 mM. La phase aqueuse est choisie comme phase stationnaire, la phase organique comme phase mobile.

### c- Mise en oeuvre de la séparation

2,772 g d'extrait obtenu selon la méthode décrite ci-dessus sont dissous dans 20 ml dé méthanol. La solution est acidifiée (contrôle au papier pH) par de l'acide méthane-sulfonique afin d'ioniser les espèces alcaloïdiques. Le méthanol est ensuite évaporé sous pression réduite, puis le résidu sirupeux est dissous dans 20 ml de phase stationnaire aqueuse acidifiée et 1 ml de phase mobile organique neutre.

La colonne est remplie de phase stationnaire (300 tours/min, mode ascendant, 20 ml/min), puis la rotation est fixée à 1700 tours/min. Le volume d'injection est alors introduit dans la colonne via une boucle d'injection, poussé par la phase mobile à 8 ml/min en mode ascendant. L'élution est poursuivie pendant 2 heures, le pourcentage de rétention de la phase stationnaire étant de 70% et la perte de charge de 68 bars. Aucune fuite n'est observée. L'effluent est fractionné grâce à un collecteur automatique de fractions (SuperFrac Pharmacia). La détection s'effectue en UV (254 nm), et par un suivi en ligne du pH. La galanthamine est éluée entre les fractions 62 et 100. Après évaporation sous pression réduite des solvants, on obtient 1,219g de galanthamine base (44% de l'extrait). La pureté avant recristallisation a été estimée supérieure à 99% par chromatographie sur couche mince (CCM), CLHP puis par RMN ¹H et ¹³C (Bruker DRX 500 MHz).

D'autres composés minoritaires ont été également isolés. Il s'agit de : la narwédine, la norgalanthamine et de l'ungiminorine.

### EXEMPLE 2 - Mode descendant Leucojum aestivum

### a- Système biphasique de solvants:

Le système utilisé correspond au mélange toluène/heptane/acétone/eau 24:8:10:34 (v/v). Les solvants sont agités dans une ampoule à décanter, puis séparés. La phase aqueuse est acidifiée par de l'acide méthane-sulfonique (déplaceur) à une concentration de 8 mM. La phase organique est alcalinisée par de la triéthylamine (reteneur) à une concentration de 10 mM. La phase aqueuse est choisie comme phase mobile, la phase organique comme phase stationnaire.

### b- Séparation par CPC

2,6 g d'extrait obtenu selon la méthode décrite ci-dessus sont dissous dans 20 ml de phase stationnaire organique alcalinisée et 1 ml de phase mobile aqueuse neutre.

La colonne est remplie de phase stationnaire organique (300 tours/min, mode ascendant, 20 ml/min), puis la rotation est fixée à 1700 tours/min. Le volume d'injection est alors introduit dans la colonne *via* une boucle d'injection puis poussé par la phase mobile à 8 ml/min en mode descendant. L'élution est poursuivie pendant 2 heures, le pourcentage de rétention de la phase stationnaire étant de 65% et la perte de charge de 67 bars. Aucune fuite de phase stationnaire n'est observée. L'effluent est fractionné grâce à un collecteur automatique de fractions (SuperFrac Pharmacia). La détection s'effectue en UV (254 nm), et par un suivi en ligne du pH. L'analyse des fractions par CCM permet de détecter celles contenant la galanthamine. Elles sont rassemblées, puis évaporées sous pression réduite. On obtient 1,142g de galanthamine base (44% de l'extrait). La pureté avant recristallisation a été estimée supérieure à 99% par CCM, CLHP puis par RMN ¹H et ¹³C (Bruker DRX 500 MHz).

### EXEMPLE-3 - Mode ascendant Narcissus Carlton

### a- Système biphasique de solvants :

Le système utilisé correspond au mélange éther de méthyle et de tertiobutyle/acétonitrile/ eau 4:1:5 (v/v). Les solvants sont agités dans une ampoule à décanter, puis séparés. La phase aqueuse est acidifiée par de l'acide méthane-sulfonique (reteneur) à une concentration de 20 mM. La phase organique est alcalinisée par de la triéthylamine (déplaceur) à une concentration de 16 mM. La phase aqueuse est choisie comme phase stationnaire, la phase organique comme phase mobile.

### b- Séparation par CPC

5g d'extrait alcaloïdique de bulbes de *Narcissus Carlton* obtenu selon la méthode décrite ci-dessus sont dissous dans 20 ml de méthanol. La solution est acidifiée à pH environ 3 (contrôle au papier pH) par de l'acide methane-sulfonique afin d'ioniser les espèces alcaloïdiques. Le méthanol est ensuite évaporé sous pression réduite, puis le résidu sirupeux est dissous dans 20 ml de phase stationnaire aqueuse acidifiée et 1 ml de phase mobile organique neutre.

La colonne est remplie de phase stationnaire (300 tours/min, mode ascendant, 20 ml/min), puis la rotation est fixée à 1200 tours/min. Le volume d'injection est alors introduit dans la colonne via une boucle d'injection, poussé par la phase mobile à 8 ml/min en mode ascendant. L'élution est poursuivie pendant 3 heures, le pourcentage de rétention de la phase stationnaire étant de 66% et la perte de charge de 42 bars. Aucune fuite de phase stationnaire n'est observée. L'effluent est fractionné grâce à un collecteur automatique de fractions (SuperFrac Pharmacia). La détection s'effectue en UV (254 nm), et par un suivi en ligne du pH. La galanthamine est éluée entre 80 et 135 minutes. Après évaporation sous pression réduite des solvants, on obtient 2,031 g de galanthamine base (forme amine). La pureté avant recristallisation a été estimée supérieure à 99% par CCM, CLHP puis par RMN ¹H et ¹³C (Bruker DRX 500 MHz).

### EXEMPLE 4 - Mode descendant Narcissus Carlton

### a- Système biphasique de solvants:

Le système utilisé correspond au mélange éther de méthyle et de tertiobutyle/acétonitrile/ eau 4:1:5 (v/v). Les solvants sont agités dans une ampoule à décanter, puis séparés. La phase aqueuse est acidifiée par de l'acide méthane-sulfonique (déplaceur) à une concentration de 16 mM. La phase organique est alcalinisée par de la triéthylamine (reteneur) à une concentration de 20 mM. La phase aqueuse est choisie comme phase mobile, la phase organique comme phase stationnaire.

### b- Séparation par CPC

5 g d'extrait alcaloïdique de bulbes de *Narcissus Carlton* obtenu selon la méthode décrite ci-dessus sont dissous dans 20 ml de phase stationnaire organique alcalinisée et 1 ml de phase mobile aqueuse neutre.

La colonne est remplie de phase stationnaire (300 tours/min, mode ascendant, 20 ml/min), puis la rotation est fixée à 1200 tours/min. Le volume d'injection est alors introduit dans la colonne *via* une boucle d'injection, poussé par la phase mobile à 8 ml/min (mode descendant). L'élution est poursuivie pendant 2 heures, le pourcentage de rétention de la phase stationnaire étant de 61% et la perte de charge de 47 bars. Aucune fuite de phase stationnaire n'est observée. L'effluent est fractionné grâce à un collecteur automatique de fractions (SuperFrac Pharmacia). La détection s'effectue en UV (254 nm), et par un suivi en ligne du pH. La galanthamine est éluée entre 71 et 127 minutes. Après évaporation sous pression réduite des solvants, on obtient 2,650g de galanthamine sous forme de méthanesulfonate. La pureté avant recristallisation a été estimée supérieure à 99% par CCM, CLHP puis par RMN ¹H et ¹³C (Bruker DRX 500 MHz).

### EXEMPLE 5 - Mode ascendant Narcissus Carlton (sans reteneur)

### a- Système biphasique de solvants :

Le système utilisé correspond au mélange éther de méthyle et de tertiobutyle/acétonitrile/ eau 4:1:5 (v/v). Les solvants sont agités dans une ampoule à décanter, puis séparés. La phase organique est alcalinisée par de la triéthylamine à une concentration de 16 mM. La phase organique reste neutre. La phase aqueuse est choisie comme phase stationnaire, la phase organique comme phase mobile.

### b- Séparation par CPC

5 g d'extrait alcaloïdique de bulbes de *Narcissus Carlton* obtenu selon la méthode décrite ci-dessus sont dissous dans 20 ml de méthanol. La solution est acidifiée à pH environ 3 (contrôle au papier pH) par de l'acide methane-sulfonique afin d'ioniser les espèces alcaloïdiques. Le méthanol est ensuite évaporé sous pression réduite, puis le résidu sirupeux est dissous dans 20 ml de phase stationnaire aqueuse acidifiée et 1 ml de phase mobile organique neutre.

La colonne est remplie de phase stationnaire (300 tours/min, mode ascendant, 20 ml/min), puis la rotation est fixée à 1200 tours/min. Le volume d'injection est alors introduit dans la colonne via une boucle d'injection, poussé par la phase mobile à 8 ml/min. L'élution est poursuivie pendant 110 minutes, le pourcentage de rétention de la phase stationnaire étant de 67% et la perte de charge de 41 bars. Aucune fuite de phase stationnaire n'est observée. L'effluent est fractionné grâce à un collecteur automatique de fractions (SuperFrac Pharmacia). La détection s'effectue en UV (254 nm), et par un suivi en ligne du pH. La galanthamine est éluée entre 45 et 100 minutes. Après évaporation sous pression réduite des solvants, on obtient 1,660 g de galanthamine base. La pureté avant recristallisation a été estimée supérieure à 99% par CCM, CLHP puis par RMN ¹H et ¹³C (Bruker DRX 500 MHz).

### RÉFÉRENCES

- Berthod, A. (2002) Ed. Countercurrent chromatography - The support-free liquid phase, Elsevier Science B.V.,
- Foucault, A.P. (1994) Ed. Centrifugal Partition Chromatography - Theory and Practice, Marcel Dekker, New York,
- Intes, O., Renault, J.H., Sinquin, C., Zèches-Hanrot, M., Nuzillard, J.M. (2001) J. Chromatography A., 918, 47-57,
- Ito, Y. (1995) Modern Countercurrent Chromatography,
- Ito, Y., Ma, Y. (1996) J. Chromatography A., 753, 1-36,
- Maciuk; A, Renault, J.H., Margraff, R., Trébuchet, P., Zèches-Hanrot, M., Nuzillard, J.M. (2004) Anal. Chem., 76, 6179-6186;
- Marchal L., Intes, O., Foucault, A., Legrand, J., Nuzillard, J.M., Renault, J.H. (2003) J. Chromatography A., 1005, 51-62,
- Margraff, R. (1994) Ed. Centrifugal Partition Chromatography, Chromatographic Sciences Series, vol. 68, Marcel Dekker, New York, ch. 12, p. 331-353,
- Renault, J.H., Nuzillard, J.M., Le Crouéour, G., Thépenier, P., Zèches-Hanrot, M., Le Men-Oliver, L. (1999) J. Chromatography A., 849, 421-431,
- Renault, J.H., Nuzillard, J.M., Intes, O., Maciuk, A. (2002) Countercurrent chromatography, Comprehensive Analytical Chemistry, vol. 38, Elsevier, Amsterdam, ch. 3, p. 49-83,
- Tiselius, A. (1943) Arkiv for Kemi och Geologo, 16A, 1-11,
- Weisz, A., Scher, A., Shinomiya, K., Fales, H.M., Ito, Y. (1994) J. Am. Chem. Soc., 116, 704-708.

## Revendications

1. Utilisation d'un procédé de chromatographie de partage centrifuge en mode déplacement, pour la mise en oeuvre d'un procédé de purification de la galanthamine ou de ses dérivés, à partir d'une composition de départ, contenant au moins 20% de galanthamine ou de ses dérivés, ledit procédé comprenant une étape de centrifugation d'une combinaison d'au moins deux solvants et de ladite composition de départ, pendant un temps suffisant pour purifier la galanthamine ou ses dérivés,
lesdits solvants étant tels qu'ils forment deux phases non miscibles, à savoir une phase aqueuse et une phase organique, la phase aqueuse servant de phase mobile ou de phase stationnaire, et la phase organique servant respectivement de phase stationnaire ou de phase mobile, la phase mobile contenant un déplaceur qui est un acide ou une base.

2. Procédé de purification de la galanthamine ou de ses dérivés à partir d'une composition de départ, contenant au moins 20% de galanthamine ou de ses dérivés, par chromatographie de partage centrifuge en mode déplacement, ledit procédé comprenant une étape de centrifugation
- d'une combinaison d'au moins deux solvants, et de préférence d'au moins trois solvants, et
- de ladite composition de départ
pendant un temps suffisant pour purifier la galanthamine ou ses dérivés,
lesdits solvants étant tels qu'ils forment deux phases non miscibles, à savoir une phase aqueuse et une phase organique, la phase aqueuse servant de phase mobile ou de phase stationnaire, et la phase organique servant respectivement de phase stationnaire ou de phase mobile, la phase mobile contenant un déplaceur qui est un acide ou une base.

3. Procédé selon la revendication 2, **caractérisé en ce que** la composition de départ est un extrait de plante ou de la matière biologique produisant de la galanthamine ou ses dérivés, telle que des cellules de plantes, ou un mélange de composés obtenu par synthèse organique contenant notamment de la galanthamine et/ou ses dérivés, ledit extrait de plante ou ladite matière biologique ou ledit mélange étant dissous dans la phase mobile ou la phase stationnaire.

4. Procédé selon la revendication 2 ou 3, **caractérisé en ce que** les deux phases liquides non miscibles correspondent à une combinaison d'au moins deux solvants, à savoir de l'eau et un solvant non miscible ou partiellement miscible à l'eau, et forment ainsi une phase aqueuse et une phase organique.

5. Procédé selon l'une des revendications 2 à 4, **caractérisé en ce que** les deux phases liquides non miscibles correspondent à un mélange d'au moins trois solvants, à savoir de l'eau, un solvant non miscible ou partiellement miscible à l'eau et un "solvant pont", ledit solvant pont étant un solvant partiellement ou totalement soluble dans l'eau et dans le solvant non miscible ou partiellement miscible à l'eau,
lesdits solvants étant tels qu'ils forment un système biphasique comprenant une phase aqueuse et une phase organique.

6. Procédé selon la revendication 4 ou 5, **caractérisé en ce que** le solvant non miscible ou partiellement miscible à l'eau est choisi parmi :
- les éthers tels que l'éther de méthyle et de tertiobutyle (MTBE) et l'éther d'éthyle et de tertiobutyle,
- les cétones non miscibles à l'eau telles que la méthyléthylcétone (MEK) et la méthylisobutylcétone (MIBK),
- les hydrocarbures aromatiques tels que le toluène,
- les hydrocarbures aliphatiques tels que l'hexane, l'heptane et les cyclohexanes,
- les éthers de pétrole,
- les alcools lourds dont la chaîne carbonée comprend au moins 4 atomes de carbone, tels que le n-butanol, le 2-butanol et l'isobutanol,
- les siloxanes non miscibles à l'eau tels que l'hexaméthyldisiloxane,
- les solvants halogénés non miscibles à l'eau, tels que le chloroforme, le dichlorométhane et le dichloro-1,2-éthane, ou
- les esters tels que l'acétate d'éthyle et l'acétate de butyle.

7. Procédé selon la revendication 5, **caractérisé en ce que** le solvant "pont" est choisi parmi : les alcools légers dont la chaîne carbonée comprend moins de 4 atomes de carbone tels que le méthanol, l'éthanol, les propanols, l'acétonitrile, l'acétone, le tétrahydrofuranne, le diméthylsulfoxyde et le diméthylformamide.

8. Procédé selon l'une des revendications 2 à 7, **caractérisé en ce que** la phase stationnaire correspond à la phase aqueuse et la phase mobile correspond respectivement à la phase organique, et **en ce que** le déplaceur contenu dans la phase mobile organique est une base.

9. Procédé selon l'une des revendications 2 à 7, **caractérisé en ce que** la phase stationnaire correspond à la phase organique et la phase mobile correspond respectivement à la phase aqueuse, et **en ce que** le déplaceur contenu dans la phase mobile aqueuse est un acide.

10. Procédé selon l'une des revendications 2 à 9, **caractérisé en ce qu'**un reteneur est :
. soit ajouté dans la composition de départ ou dans la phase stationnaire,
. soit est un élément de la composition de départ,
ledit reteneur étant un acide ou une base.

11. Procédé selon la revendication 8, **caractérisé en ce qu'**on ajoute dans la composition de départ ou dans la phase stationnaire un reteneur, qui est un acide.

12. Procédé selon la revendication 9, **caractérisé en ce qu'**on ajoute dans la composition de départ ou dans la phase stationnaire un reteneur, qui est une base.

13. Procédé selon l'une des revendications 2 à 12, comprenant les étapes suivantes :
- l'injection de la phase stationnaire dans une colonne de chromatographie de partage centrifuge, ladite phase stationnaire contenant un reteneur tel que défini dans la revendication 10, afin d'obtenir une colonne de chromatographie de partage centrifuge remplie de phase stationnaire,
- l'injection de la composition de départ telle que définie dans l'une des revendications 2 à 12 dans la colonne de chromatographie de partage centrifuge remplie de phase stationnaire, pour obtenir une colonne de chromatographie de partage centrifuge chargée avec ladite phase stationnaire et ladite composition de départ, et
- l'introduction par pompage dans la colonne telle qu'obtenue à l'étape précédente, de la phase mobile dans laquelle un déplaceur est ajouté telle que définie dans la revendication 8 ou 9, afin d'éluer la galanthamine ou ses dérivés.

14. Procédé selon l'une des revendications 1 à 13, **caractérisé en ce que** le déplaceur est choisi parmi :
- les acides minéraux tels que HCl ou H₂SO₄,
- les acides organiques tels que l'acide méthane-sulfonique, l'acide trifluoroacétique, l'acide acétique, l'acide tartrique ou l'acide citrique,
- les bases minérales telles que l'ammoniac ou la soude, ou
- les bases organiques telles que la triéthylamine.

15. Procédé selon l'une des revendications 10 à 14, **caractérisé en ce que** le reteneur est choisi parmi :
- les bases minérales telles que l'ammoniac ou la soude, ou
- les bases organiques telles que la triéthylamine.
- les acides minéraux tels que HCl ou H₂SO₄,
- les acides organiques tels que l'acide méthane-sulfonique, l'acide trifluoroacétique, l'acide acétique, l'acide tartrique ou l'acide citrique,

16. Procédé selon l'une des revendications 2 à 15, comprenant l'utilisation d'une combinaison des solvants suivants :
• toluène, heptane, acétone et eau, ou
• éther de méthyle et de tertiobutyle, acétonitrile et eau, ou
• éther de méthyle et de tertiobutyle, acétone et eau, ou
• méthylisobutylcétone, acétone et eau.

17. Procédé selon la revendication 16, comprenant l'utilisation de la combinaison suivante de solvants : toluène, heptane, acétone et eau, dans des proportions volumiques telles que :
- le pourcentage volumique du toluène est supérieur à celui de l'heptane,
- le pourcentage volumique de l'acétonitrile ne dépasse pas 50%, et
- le mélange de ces solvants est biphasique,
et notamment dans les proportions volumiques 24:8:10:34.

18. Procédé selon la revendication 16, comprenant l'utilisation de la combinaison suivante de solvants : éther de méthyle et de tertiobutyle, acétonitrile et eau, dans des proportions volumiques telles que :
- le pourcentage volumique de l'acétonitrile ne dépasse pas 45%, et
- le mélange de ces solvants est biphasique,
et notamment dans les proportions volumiques 4:1:5.

19. Procédé selon l'une des revendications 2 à 18, **caractérisé en ce que** la composition de départ est un extrait de parties aériennes ou de bulbes d'une Amaryllidaceae, notamment des genres *Leucojum, Narcissus* ou *Galanthus,* et est de préférence un extrait de feuilles de *Leucojum aestivum* ou un extrait de bulbes de *Narcissus carlton.*

20. Procédé selon l'une des revendications 2 à 19, **caractérisé en ce que** la composition de départ est un extrait de feuilles de *Leucojum aestivum* et **en ce que** la combinaison de solvants est la suivante : toluène, heptane, acétone et eau, dans les proportions volumiques 24:8:10:34.

21. Procédé selon l'une des revendications 2 à 19, **caractérisé en ce que** la composition de départ est un extrait de bulbes de *Narcissus carlton* et **en ce que** la combinaison de solvants est la suivante : éther de méthyle et de tertiobutyle, acétonitrile et eau, dans les proportions volumiques 4:1:5.

22. Procédé selon la revendication 11, **caractérisé en ce qu'**il comprend les étapes suivantes :
- l'injection de la phase stationnaire aqueuse dans une colonne de chromatographie de partage centrifuge, ladite phase stationnaire contenant un reteneur acide tel que défini dans la revendication 11, afin d'obtenir une colonne de chromatographie de partage centrifuge remplie de phase stationnaire acidifiée,
- l'injection de la composition de départ, dans laquelle la galanthamine ou ses dérivés sont sous forme de sels, dans la colonne de chromatographie de partage centrifuge remplie de phase stationnaire acidifiée, pour obtenir une colonne de chromatographie de partage centrifuge chargée avec ladite phase stationnaire acidifiée et ladite composition de départ, et
- l'introduction par pompage de la phase mobile organique à travers la colonne telle qu'obtenue à l'étape précédente, dans laquelle un déplaceur basique est ajouté, afin d'éluer la galanthamine ou ses dérivés sous forme basique.

23. Procédé selon la revendication 22, **caractérisé en ce que** le reteneur acide est ajouté dans la phase stationnaire.

24. Procédé selon la revendication 12, **caractérisé en ce qu'**il comprend les étapes suivantes :
- l'injection de la phase stationnaire organique dans une colonne de chromatographie de partage centrifuge, ladite phase stationnaire contenant un reteneur basique tel que défini dans la revendication 12, afin d'obtenir une colonne de chromatographie de partage centrifuge remplie de phase stationnaire alcalinisée,
- l'injection de la composition de départ, dans laquelle la galanthamine ou ses dérivés sont sous forme basique, dans la colonne de chromatographie de partage centrifuge remplie de phase stationnaire alcalinisée, pour obtenir une colonne de chromatographie de partage centrifuge chargée avec ladite phase stationnaire alcalinisée et ladite composition de départ, et
- l'introduction par pompage de la phase mobile aqueuse à travers la colonne telle qu'obtenue à l'étape précédente, dans laquelle un déplaceur acide est ajouté, afin d'éluer la galanthamine ou ses dérivés sous forme de sels.

25. Procédé selon la revendication 24, **caractérisé en ce que** le reteneur basique est ajouté dans la phase stationnaire.

## Claims

1. Use of a process of centrifugal partition chromatography in displacement mode, for the implementation of a process for the purification of galanthamine or its derivatives, from a starting composition, containing at least 20% of galanthamine or its derivatives, said process comprising a stage of centrifuging a combination of at least two solvents and said starting composition, for a sufficient time to purify galanthamine or its derivatives,
said solvents being such that they form two non-miscible phases, namely an aqueous phase and an organic phase, the aqueous phase serving as the mobile phase or the stationary phase, and the organic phase serving respectively as the stationary phase or the mobile phase, the mobile phase containing an acidic or basic displacing agent.

2. Process for the purification of galanthamine or its derivatives from a starting composition, containing at least 20% of galanthamine or its derivatives, by centrifugal partition chromatography in displacement mode, said process comprising a stage of centrifuging
- a combination of at least two solvents, and preferably of at least three solvents, and
- said starting composition
for a sufficient time to purify galanthamine or its derivatives,
said solvents being such that they form two non-miscible phases, namely an aqueous phase and an organic phase, the aqueous phase serving as the mobile phase or the stationary phase, and the organic phase serving respectively as the stationary phase or the mobile phase, the mobile phase containing an acidic or basic displacing agent.

3. Process according to claim 2, **characterized in that** the starting composition is a plant extract or biological material producing galanthamine or its derivatives, such as plant cells, or a mixture of compounds obtained by organic synthesis containing in particular galanthamine and/or its derivatives, said plant extract or said biological material or said mixture being dissolved in the mobile phase or the stationary phase.

4. Process according to claim 2 or 3, **characterized in that** the two non-miscible liquid phases correspond to a combination of at least two solvents, namely water and a solvent which is non-miscible or partially miscible with water, thus forming an aqueous phase and an organic phase.

5. Process according to one of claims 2 to 4, **characterized in that** the two non-miscible liquid phases correspond to a mixture of at least three solvents, namely water, a solvent which is non-miscible or partially miscible with water and a "bridge solvent", said bridge solvent being a solvent partially or totally soluble in water and in the solvent which is non-miscible or partially miscible with water,
said solvents being such that they form a biphasic system comprising an aqueous phase and an organic phase.

6. Process according to claim 4 or 5, **characterized in that** the solvent which is non-miscible or partially miscible with water is chosen from:
- ethers such as methyl tert-butyl ether (MTBE) and ethyl tert-butyl ether,
- ketones which are non-miscible with water such as methyl ethyl ketone (MEK) and the methyl isobutyl ketone (MIBK),
- aromatic hydrocarbons such as toluene,
- aliphatic hydrocarbons such as hexane, heptane and the cyclohexanes,
- petroleum ethers,
- heavy alcohols the carbon-containing chain of which comprises at least 4 carbon atoms, such as n-butanol, 2-butanol and isobutanol,
- siloxanes which are non-miscible with water such as hexamethyldisiloxane,
- halogenated solvents which are non-miscible with water, such as chloroform, dichloromethane and dichloro-1,2-ethane, or
- esters such as ethyl acetate and butyl acetate.

7. Process according to claim 5, **characterized in that** the "bridge" solvent is chosen from: light alcohols the carbon-containing chain of which comprises less than 4 carbon atoms such as methanol, ethanol, propanols, acetonitrile, acetone, tetrahydrofuran, dimethylsulphoxide and dimethylformamide.

8. Process according to one of claims 2 to 7, **characterized in that** the stationary phase corresponds to the aqueous phase and the mobile phase corresponds respectively to the organic phase, and **in that** the displacing agent contained in the organic mobile phase is a base.

9. Process according to one of claims 2 to 7, **characterized in that** the stationary phase corresponds to the organic phase and the mobile phase corresponds respectively to the aqueous phase, and **in that** the displacing agent contained in the aqueous mobile phase is an acid.

10. Process according to one of claims 2 to 9, **characterized in that** a retaining agent is:
. either added in the starting composition or in the stationary phase,
. or is an element of the starting composition,
said retaining agent being an acid or a base.

11. Process according to claim 8, **characterized in that** a retaining agent, which is an acid, is added in the starting composition or in the stationary phase.

12. Process according to claim 9, **characterized in that** a retaining agent, which is a base, is added in the starting composition or in the stationary phase.

13. Process according to one of claims 2 to 12, comprising the following stages:
- the injection of the stationary phase into a centrifugal partition chromatography column, said stationary phase containing a retaining agent as defined in claim 10, in order to obtain a centrifugal partition chromatography column filled with stationary phase,
- the injection of the starting composition as defined in one of claims 2 to 12 into the centrifugal partition chromatography column filled with stationary phase, in order to obtain a centrifugal partition chromatography column loaded with said stationary phase and said starting composition, and
- the introduction by pumping into the column as obtained in the previous stage, of the mobile phase in which a displacing agent is added as defined in claim 8 or 9, in order to elute galanthamine or its derivatives.

14. Process according to one of claims 1 to 13, **characterized in that** the displacing agent is chosen from:
- mineral acids such as HCl or H₂SO₄,
- organic acids such as methanesulphonic acid, trifluoroacetic acid, acetic acid, tartaric acid or citric acid,
- mineral bases such as ammonia or soda, or
- organic bases such as the triethylamine.

15. Process according to one of claims 10 to 14, **characterized in that** the retaining agent is chosen from:
- mineral bases such as ammonia or soda, or
- organic bases such as triethylamine.
- mineral acids such as HCl or H₂SO₄,
- organic acids such as methanesulphonic acid, trifluoroacetic acid, acetic acid, tartaric acid or citric acid,

16. Process according to one of claims 2 to 15, comprising the use of a combination of the following solvents:
• toluene, heptane, acetone and water, or
• methyl tert-butyl ether, acetonitrile and water, or
• methyl tert-butyl ether, acetone and water, or
• methyl isobutyl ketone, acetone and water.

17. Process according to claim 16, comprising the use of the following combination of solvents: toluene, heptane, acetone and water, in volume proportions such that:
- the volume percentage of the toluene exceeds that of the heptane,
- the volume percentage of the acetonitrile does not exceed 50%, and
- the mixture of these solvents is biphasic,
and in particular in the volume proportions 24:8:10:34.

18. Process according to claim 16, comprising the use of the following combination of solvents: methyl tert-butyl ether, acetonitrile and water, in volume proportions such that:
- the volume percentage of the acetonitrile does not exceed 45%, and
- the mixture of these solvents is biphasic,
and in particular in the volume proportions 4:1:5.

19. Process according to one of claims 2 to 18, **characterized in that** the starting composition is an extract of aerial parts or bulbs of Amaryllidaceae, in particular of the genus *Leucojum, Narcissus* or *Galanthus,* and is preferably an extract of leaves of *Leucojum aestivum* or an extract of bulbs of *Narcissus carlton.*

20. Process according to one of claims 2 to 19, **characterized in that** the starting composition is an extract of leaves of *Leucojum aestivum* and **in that** the combination of solvents is as follows: toluene, heptane, acetone and water, in the volume proportions 24:8:10:34.

21. Process according to one of claims 2 to 19, **characterized in that** the starting composition is an extract of bulbs of *Narcissus carlton* and **in that** the combination of solvents is as follows: methyl tert-butyl ether, acetonitrile and water, in the volume proportions 4:1:5.

22. Process according to claim 11, **characterized in that** it comprises the following stages:
- the injection of the aqueous stationary phase into a centrifugal partition chromatography column, said stationary phase containing an acidic retaining agent as defined in claim 11, in order to obtain a centrifugal partition chromatography column filled with acidified stationary phase,
- the injection of the starting composition, in which galanthamine or its derivatives are in the form of salts, into the centrifugal partition chromatography column filled with acidified stationary phase, in order to obtain a centrifugal partition chromatography column loaded with said acidified stationary phase and said starting composition, and
- the introduction by pumping of the organic mobile phase through the column as obtained in the previous stage, in which a basic displacing agent is added, in order to elute galanthamine or its derivatives in basic form.

23. Process according to claim 22, **characterized in that** the acidic retaining agent is added in the stationary phase.

24. Process according to claim 12, **characterized in that** it comprises the following stages:
- the injection of the organic stationary phase into a centrifugal partition chromatography column, said stationary phase containing a basic retaining agent as defined in claim 12, in order to obtain a centrifugal partition chromatography column filled with alkalinized stationary phase,
- the injection of the starting composition, in which galanthamine or its derivatives are in basic form, into the centrifugal partition chromatography column filled with alkalinized stationary phase, in order to obtain a centrifugal partition chromatography column loaded with said alkalinized stationary phase and said starting composition, and
- the introduction by pumping of the aqueous mobile phase through the column as obtained in the previous stage, in which an acidic displacing agent is added, in order to elute galanthamine or its derivatives in the form of salts.

25. Process according to claim 24, **characterized in that** the basic retaining agent is added in the stationary phase.

## Patentansprüche

1. Verwendung eines Verfahrens der zentrifugalen Verteilungschromatographie im Verdrängungsmodus zum Durchführen eines Verfahrens zur Reinigung von Galanthamin oder Galanthamindderivaten, ausgehend von einer Ausgangszusammensetzung, die mindestens 20 % Galanthamin oder Galanthamindderivate enthält, wobei das Verfahren einen Schritt des Zentrifugierens einer Kombination aus mindestens zwei Lösemitteln und dieser Ausgangszusammensetzung umfasst, während eines Zeitraums, der ausreicht, um das Galanthamin oder die Galanthaminderivate zu reinigen, wobei die Lösemittel derart sind, dass sie zwei nicht mischbare Phasen bilden, nämlich eine wässrige Phase und eine organische Phase, wobei die wässrige Phase als mobile Phase oder als stationären Phase dient, und die organische Phase entsprechend als stationäre Phase bzw. mobile Phase dient, wobei die mobile Phase ein Verdrängungsmittel enthält, das eine Säure oder eine Base ist.

2. Verfahren zur Reinigung von Galanthamin oder Galanthamindderivaten ausgehend von einer Ausgangszusammensetzung, die mindestens 20 % Galanthamin oder Galanthamindderivate enthält, durch zentrifugale Verteilungschromatographie im Verdrängungsmodus, wobei das Verfahren einen Schritt des Zentrifugierens
- einer Kombination aus mindestens zwei Lösemitteln, und bevorzugt mindestens drei Lösemitteln und
- der Ausgangszusammensetzung während eines Zeitraums umfasst, der ausreicht, um das Galanthamin oder die Galanthamindderivate zu reinigen,
wobei die Lösemittel derart sind, dass sie zwei nicht mischbare Phasen bilden, nämlich eine wässrige Phase und eine organische Phase, wobei die wässrige Phase als mobile Phase oder als stationäre Phase dient, und die organische Phase entsprechend als stationäre Phase bzw. als mobile Phase dient, wobei die mobile Phase ein Verdrängrngsmittel enthält, das eine Säure oder eine Base ist.

3. Verfahren nach Anspruch 2, **dadurch gekennzeichnet, dass** die Ausgangszusammensetzung ein Pflanzenextrakt oder ein biologisches Material ist, der (das) Galanthamin oder Galanthamindderivate produziert, wie etwa Pflanzenzelle oder ein durch organische Synthese erhaltenes Verbindungsgemisch, das insbesondere Galanthamin und/oder Galanthamindderivate enthält, wobei der Pflanzenextrakt oder das biologische Material oder das Gemisch in der mobilen Phase oder der stationären Phase gelöst sind.

4. Verfahren nach Anspruch 2 oder 3, **dadurch gekennzeichnet, dass** die zwei nicht mischbaren, flüssige Phasen einer Kombination aus mindestens zwei Lösemitteln entsprechen, nämlich Wasser und einem Lösemittel, das nicht oder teilweise mit Wasser mischbar ist, und so eine wässrige Phase und eine organische Phase bilden.

5. Verfahren nach einem der Ansprüche 2 bis 4, **dadurch gekennzeichnet, dass** die beiden nicht mischbaren flüssigen Phasen einem Gemisch aus mindestens drei Lösemitteln entsprechen, nämlich Wasser, einem Lösemittel, das nicht oder teilweise mit Wasser mischbar ist, und einem "Brückenlösemittel", wobei das Brückenlösemittel ein Lösemittel ist, das teilweise oder vollständig in wasser und dem Lösemittel löslich ist, das nicht oder teilweise mit Wasser mischbar ist, wobei die Lösemittel derart sind, dass sie ein zweiphasiges System bilden, das eine wässrige Phase und ein organische Phase umfasst.

6. Verfahren nach Anspruch 4 oder 5, **dadurch gekennzeichnet, dass** das Lösemittel, das nicht oder teilweise mit Wasser mischbar ist, ausgewählt ist aus:
- Ethern, wie etwa tert-Butylmethylether (MTBE) und tert-Butylethylether,
- Ketonen, die nicht mit Wasser mischbar sind, wie etwa Methylethylketon (MEK) und Methylisobutylketon (MIBK),
- aromatischen Kohlenwasserstoffen, wie etwa Toluen,
- aliphatischen Kohlenwasserstoffen, wie etwa Hexan, Heptan und den Cyclohexanen,
- Petrolethern,
- schweren Alkoholen, deren Kohlenstoffkette mindestens 4 Kohlenstoffatome umfasst, wie etwa n-Butancl, 2-Butanol und Isobutanol,
- Siloxanen, die nicht mit Wasser mischbar sind, wie etwa Hexamethyldisiloxan,
- halogenierten Lösemitteln, die nicht mit Wasser mischbar sind, wie etwa Chloroform, Dichlormethan und Dichlor-1,2-ethan, oder
- Estern, wie etwa Ethylacetat und Butylacetat.

7. Verfahren nach Anspruch 5, **dadurch gekennzeichnet, dass** das "Brückenlösemittel" ausgewählt ist aus: leichten Alkoholen, deren Kohlenstoffkette mindestens 4 Kohlenstoffatome umfasst, wie etwa Methanol, Ethanol, Propanole, Acetonitril, Aceton, Tetrahydrofuran, Dimethylsulfoxid und Dimethylformamid.

8. Verfahren nach einem der Ansprüche 2 bis 7, **dadurch gekennzeichnet, dass** die stationäre Phase der wässrigen Phase entspricht und die mobile Phase entsprechend der organischen Phase entspricht, und **dadurch**, dass das Verdrängungsmittel, das in der organischen mobilen Phase enthalten ist, eine Base ist.

9. Verfahren nach einem der Ansprüche 2 bis 7, **dadurch gekennzeichnet, dass** die stationäre Phase der organischen Phase entspricht und die mobile Phase entsprechend der wässrigen Phase entspricht, und **dadurch**, dass das Verdrängungsmittel, das in der wässrigen mobilen Phase enthalten ist, eine Säure ist.

10. Verfahren nach einem der Ansprüche 2 bis 9, **dadurch gekennzeichnet, dass** ein Retentionsmittel:
• entweder der Ausgangszusammensetzung oder der stationären Phase zugegeben wird
• oder ein Element der Ausgangszusammensetzung ist,
wobei das Retentionsmittel eine Säure oder eine Base ist.

11. Verfahren nach Anspruch 8, **dadurch gekennzeichnet, dass** der Ausgangzusammensetzung oder der stationären Phase ein Retentionsmittel zugegeben wird, das eine Säure ist.

12. Verfahren nach Anspruch 9, **dadurch gekennzeichnet, dass** der Ausgangzusammensetzung oder der stationären Phase ein Retentionsmittel zugegeben wird, das eine Base ist.

13. Verfahren nach einem der Ansprüche 2 bis 12, das die folgenden Schritte umfasst:
- Injizieren der stationären Phase in eine zentrifugale Verteilungschromatographiesäule, wobei die stationäre Phase ein Retentionsmittel enthält, wie in Anspruch 10 definiert, um eine zentrifugale Verteilungschromatographiesäule zu erhalten, die mit der stationären Phase gefüllt ist,
- Injizieren der Ausgangszusammensetzung, wie in einem der Ansprüche 2 bis 12 definiert, in die zentrifugale Verteilungschromatographiesäule, die mit der stationären Phase gefüllt ist, um eine zentrifugale Verteilungschromatographiesäule zu erhalten, die mit der stationären Phase und der Ausgangszusammensetzung beladen ist, und
- Einführen durch Pumpen der mobilen Phase, der ein Verdrängungsmittel zugegeben wird, wie in Anspruch 8 oder 9 definiert, in die im vorhergehenden Schritt erhaltene Säule, um das Galanthamin oder die Galanthamindderivate zu eluieren.

14. Verfahren nach einem der Ansprüche 1 bis 13, **dadurch gekennzeichnet, dass** das Verdrängungsmittel ausgewählt ist aus:
- mineralischen Säuren, wie etwa HCl oder H₂SO₄,
- organischen Säuren, wie etwa Methansulfonsäure, Trifluoressigsäure, Essigsäure, Weinsäure oder Citronensäure,
- mineralischen Basen, wie etwa Ammoniak oder Soda, oder
- organischen Basen, wie etwa Triethylamin.

15. Verfahren nach einem der Ansprüche 10 bis 14, **dadurch gekennzeichnet, dass** das Retentionsmittel ausgewählt ist aus:
- mineralischen Basen, wie etwa Ammoniak oder Soda, oder
- organischen Basen, wie etwa Triethylamin,
- mineralischen Säuren, wie etwa HCl oder H₂SO₄,
- organischen Säuren, wie etwa Methansulfonsäure, Trifluoressigsäure, Essigsäure, Weinsäure oder Citronensäure.

16. Verfahren nach einem der Ansprüche 2 bis 15, das die Verwendung einer Kombination folgender Lösemittel umfasst:
- Toluen, Heptan, Aceton und Wasser, oder
- tert-Butylmethylether, Acetonitril und Wasser, oder
- tert-Butylmethylether, Aceton und Wasser, oder
- Methylisobutylketon, Aceton und Wasser.

17. Verfahren nach Anspruch 16, das die Verwendung der folgenden Kombination von Lösemitteln umfasst: Toluen, Heptan, Aceton und Wasser, in Volumenanteilen, so dass:
- der Volumenprozentanteil des Toluens größer ist als der des Heptans,
- der Volumenprozentanteil des Acetonitrils 50 % nicht übersteigt, und
- das Gemisch aus diesen Lösemitteln zweiphasig ist,
und insbesondere in den Volumenanteilen 24:8: 10:34.

18. Verfahren nach Anspruch 16, das die Verwendung der folgenden Kombination von Lösemittel umfasst: tert-Butylmethylether, Acetonitril und Wasser in den Volumenanteilen, so dass
- der Volumenprozentanteil des Acetonitrils 45 % nicht übersteigt, und
- das Gemisch aus diesen Lösemitteln zweiphasig ist,
und insbesondere in den Volumenanteilen 4:1:5.

19. Verfahren nach einem der Ansprüche 2 bis 18, **dadurch gekennzeichnet, dass** die Ausgangszusammensetzung ein Extrakt der oberirdischen Teile oder der Zwiebeln einer Amaryllidaceae, insbesondere der Gattungen *Leucojum, Narcissus* oder *Galanthus,* und bevorzugt ein Extrakt der Blätter von *Leucojum aestivum* oder ein Extrakt der Zwiebeln von *Narcissus carlton* ist.

20. Verfahren nach einem der Ansprüche 2 bis 19, **dadurch gekennzeichnet, dass** die Ausgangszusammensetzung ein Extrakt der Blätter von *Leucojum aestivum* ist und **dadurch**, dass die Kombination der Lösemittel Folgende ist: Toluen, Heptan, Aceton und Wasser in den Volumenanteilen 24:8:10:34.

21. Verfahren nach einem der Ansprüche 2 bis 19, **dadurch gekennzeichnet, dass** die Ausgangszusammensetzung ein Extrakt der Zwiebeln von *Narcissus carlton* ist und **dadurch**, dass die Kombination der Lösemittel Folgende ist: Tert-Butylmethylether, Acetonitril und Wasser in den Volumenanteilen 4:1:5.

22. Verfahren nach Anspruch 11, **dadurch gekennzeichnet, dass** es die folgenden Schritte umfasst:
- Injizieren der wässrigen stationären Phase in eine zentrifugale Verteilungschromatographiesäule, wobei die stationäre Phase ein saures Retentionsmittel enthält, wie in Anspruch 11 definiert, um eine zentrifugale Verteilungschromatographiesäule zu erhalten, die mit der angesäuerten stationären Phase gefüllt ist,
- Injizieren der Ausgangszusammensetzung, in der das Galanthamin oder die Galanthaminderivate in Form von Salzen vorliegen, in die zentrifugale Verteilungschromatographiesäule, die mit der angesäuerten stationären Phase gefüllt ist, um eine zentrifugale Verteilungschromatographiesäule zu erhalten, die mit der angesäuerten stationären Phase und der Ausgangszusammensetzung beladen ist, und
- Einführen durch Pumpen der organischen mobilen Phase durch die Säule, wie sie im vorhergehenden Schritt erhalten wurde, wobei ein basisches Verdrängungsmittel zugegeben wird, um das Galanthamin oder die Galanthaminderivate in basischer Form zu eluieren.

23. Verfahren nach Anspruch 22, **dadurch gekennzeichnet, dass** das saure Retentionsmittel der stationären Phase zugegeben wird.

24. Verfahren nach Anspruch 12, **dadurch gekennzeichnet, dass** es die folgenden Schritte umfasst:
- Injizieren der organischen stationären Phase in eine zentrifugale Verteilungschromatographiesäule, wobei die stationäre Phase ein basisches Retentionsmittel enthält, wie in Anspruch 12 definiert, um eine zentrifugale Verteilungschromatographiesäule zu erhalten, die mit der alkalisierten stationären Phase gefüllt ist,
- Injizieren der Ausgangszusammensetzung, in der das Galanthamin oder die Galanthaminderivate in basischer Form vorliegen, in die zentrifugale Verteilungschromatographiesäule, die mit der alkalisierten stationären Phase gefüllt ist, um eine zentrifugale verteilungschromatographiesäule zu erhalten, die mit der alkalisierten stationären Phase und der Ausgangszusammensetzung beladen ist, und
- Einführen durch Pumpen der wässrigen mobilen Phase durch die Säule, wie sie im vorhergehenden Schritt erhalten wurde, wobei ein saures Verdrängungsmittel zugegeben wird, um das Galanthamin oder die Galanthaminderivate in Form von Salzen zu eluieren.

25. Verfahren nach Anspruch 24, **dadurch gekennzeichnet, dass** das basische Retentionsmittel der stationären Phase zugegeben wird.
